# EUROPEAN PATENT SPECIFICATION

(11) **EP 4 196 018 B1**
(45) Date of publication and mention of the grant of the patent: **11.11.2026**
(21) Application number: 22761613.3
(22) Date of filing: 10.08.2022
(51) Int. Cl.: A61B 17/115, A61B 17/064

(54) **CIRCULAR SURGICAL STAPLER HAVING STAPLES WITH EXPANDABLE CROWNS**
KREISFÖRMIGES CHIRURGISCHES KLAMMERGERÄT MIT KLAMMERN MIT EXPANDIERBAREN KRONEN
AGRAFEUSE CHIRURGICALE CIRCULAIRE AYANT DES AGRAFES À COURONNES EXTENSIBLES

(30) Priority: 13.08.2021 US 202117401451
(43) Date of publication of application: 21.06.2023
(73) Proprietor: Cilag GmbH International, 6300 Zug (CH)
(72) Inventor: BOUDREAUX, Chad P., Cincinnati, Ohio 45242 (US); ALDRIDGE, Jeffrey L., Cincinnati, Ohio 45242 (US); MORGAN, Nicholas M., Cincinnati, Ohio 45242 (US); STOKES, Michael J., Cincinnati, Ohio 45242 (US); KAMENIR, Marissa T., Cincinnati, Ohio 45242 (US); BRUCE, John K., Cincinnati, Ohio 45242 (US); KIMSEY, John S., Cincinnati, Ohio 45242 (US); NGUETIO TCHOUMKEU, Yvan D., Cincinnati, Ohio 45242 (US)
(74) Representative: Carpmaels & Ransford LLP
(86) International application number: PCT/IB2022/057443
(87) International publication number: WO 2023/017430

(56) References cited:
- EP-A1- 1 728 473
- EP-A1- 2 649 949

## Description

### Technical field

The present invention relates to circular surgical staplers having staples with expandable crowns.

### BACKGROUND

A circular surgical stapler may be used to form an anastomosis between two organ portions of a patient's digestive tract. Examples of circular surgical staplers are described in U.S. Pat. No. 5,292,053, entitled "Surgical Anastomosis Stapling Instrument," issued March 8, 1994; U.S. Pat. No. 5,333,773, entitled "Surgical Anastomosis Stapling Instrument," issued August 2, 1994; U.S. Pat. No. 5,350,104, entitled "Surgical Anastomosis Stapling Instrument," issued September 27, 1994; and U.S. Pat. No. 5,533,661, entitled "Surgical Anastomosis Stapling Instrument," issued July 9, 1996; and U.S. Pat. No. 8,910,847, entitled "Low Cost Anvil Assembly for a Circular Stapler," issued December 16, 2014.

Some circular staplers may include a motorized actuation mechanism. Examples of circular staplers with motorized actuation mechanisms are described in U.S. Pub. No. 2015/0083772, entitled "Surgical Stapler with Rotary Cam Drive and Return," published March 26, 2015, now abandoned; U.S. Pat. No. 9,936,949, entitled "Surgical Stapling Instrument with Drive Assembly Having Toggle Features," issued April 10, 2018; U.S. Pat. No. 9,907,552, entitled "Control Features for Motorized Surgical Stapling Instrument," issued March 6, 2018; U.S. Pat. No. 9,713,469, entitled "Surgical Stapler with Rotary Cam Drive," issued July 25, 2017; U.S. Pub. No. 2018/0132849, entitled "Staple Forming Pocket Configurations for Circular Surgical Stapler Anvil," published May 17, 2018; and U.S. Pat. No. 10,709,452, entitled "Methods and Systems for Performing Circular Stapling," issued July 14, 2020.

EP 2 649 949 A1 describes a surgical circular stapler for forming an anastomosis. The stapler has a tubular body portion, and a cartridge assembly at a distal end of the body portion. The cartridge assembly expels an annular array of staples when the stapler is fired. Each of the staples has a pair of legs and a generally bent, angled or curved back span. An anvil member at the distal end of the tubular body portion is positioned opposite the cartridge assembly to crimp the staples in tissue upon expulsion of the staples from the cartridge assembly. Hence, the anvil has a corresponding annular array of staple forming buckets. Each of the staple forming buckets has a straight configuration or a bent configuration corresponding to the staple backspan, and the staples are crimped by the buckets so that the legs extend to or beyond the back span when viewed from the side.

EP 1 728 473 A1 describes a staple for a surgical stapling instrument where the staple has an expandable back span. The staple includes a first leg extending from one end of the back span and a second leg extending from the other end of the back span. The back span defines a curve or recess to provide the staple back span with a concave profile when viewed from the side. A central portion of the back span is curved or recessed such that the central portion extends towards the penetrating tips of the staple legs. The back span of the staple need not extend in a straight line when viewed from above the back span. For example, the top profile of the back span may extend in a serpentine pattern. It is also envisioned that the profile of the staple back span when viewed from the side need not be circular but rather may have other recessed or concave configurations, e.g., U-shaped, trapezoidal, rectangular, etc.

### SUMMARY

The present invention provides an apparatus for creating an anastomosis between two lumens, according to claim 1 and its dependent claims. The apparatus comprises a body, a shaft extending distally from the body, a stapling head assembly positioned at a distal end of the shaft and an anvil. The stapling head assembly includes an annular deck member and a staple driver. The annular deck member includes a deck surface configured to contact a tissue defining a select one of the two lumens, an annular array of staple openings formed through the deck surface, wherein the annular array of staple openings is configured with a pattern having two rows of offset openings, and a plurality of staples configured to travel through the staple openings, each staple of the plurality of staples comprising a crown and a pair of legs extending from the crown, wherein the crown is angled such that each staple defines a "V" shape when viewed from the top or the bottom or the crown is curved such that each staple defines a "C" shape when viewed from the top or the bottom. Each of the plurality of staples is expandable having a first configuration and a second configuration, wherein in the second configuration a distance between the pair of legs is greater than in the first configuration. The staple driver is operable to drive the plurality of staples through the annular array of staple openings. The anvil is configured to deform the staples driven by the staple driver to form a ring-shaped staple pattern configured to connect the two lumens, wherein the staples in one row are offset from the staples in the other row and the "V" or "C" shapes defined by the staples in one row are oppositely oriented to the "V" or "C" shapes defined by adjacent staples in the other row.

### BRIEF DESCRIPTION OF THE DRAWINGS

FIG. 1 depicts a perspective view of an exemplary circular surgical stapler that includes a handle assembly, a shaft assembly, and an end effector having a stapling head assembly and an anvil;
FIG. 2 depicts a perspective view of the circular stapler of FIG. 1, with a battery pack removed from the handle assembly and the anvil separated from the stapling head assembly;
FIG. 3 depicts a perspective view of the anvil of the circular stapler of FIG. 1;
FIG. 4 depicts a perspective view of the stapling head assembly of the circular stapler of FIG. 1;
FIG. 5 depicts an exploded perspective view of the stapling head assembly of FIG. 4;
FIG. 6 depicts an exploded perspective view of the circular stapler of FIG. 1, with portions of the shaft assembly shown separated from each other;
FIG. 7A depicts a cross-sectional side view of the anvil of FIG. 3 positioned within a first section of a digestive tract and the stapling head assembly of FIG. 4 positioned within a separate second section of the digestive tract, with the anvil separated from the stapling head assembly;
FIG. 7B depicts a cross-sectional side view of the anvil of FIG. 3 positioned within the first section of the digestive tract and the stapling head assembly of FIG. 4 positioned within the separate second section of the digestive tract, with the anvil secured to the stapling head assembly;
FIG. 7C depicts a cross-sectional side view of the anvil of FIG. 3 positioned within the first section of the digestive tract and the stapling head assembly of FIG. 4 positioned within the separate second section of the digestive tract, with the anvil retracted toward the stapling head assembly to thereby clamp tissue between the anvil and the stapling head assembly;
FIG. 7D depicts a cross-sectional side view of the anvil of FIG. 3 positioned within the first section of the digestive tract and the stapling head assembly of FIG. 4 positioned within the second section of the digestive tract, with the stapling head assembly actuated to sever and staple the clamped tissue and thereby joining the first and second sections of the digestive tract;
FIG. 7E depicts a cross-sectional side view of the first and second sections of the digestive tract of FIG. 7A joined together at an end-to-end anastomosis formed with the circular stapler of FIG. 1;
FIG. 8 depicts a top view of portions of an exemplary alternate stapling head assembly for use with the instrument of FIG. 1;
FIG. 9 depicts a perspective view of portions the stapling head assembly of FIG. 8, showing an array of expandable staples in an unformed state;
FIG. 10 depicts a partial perspective view of the staples of FIG. 9, shown in a formed state.
FIG. 11 depicts a top view of portions of the stapling head assembly of FIG. 8, showing the movement of the staple line based on staple expansion;
FIG. 12 depicts a partial perspective view of the formed staples of FIG. 10, showing the movement of the staple line based on staple expansion;
FIG. 13 depicts a perspective view of an exemplary staple having a "V" shape and a spring feature;
FIG. 14A depicts a perspective view of an exemplary staple having a "V" shape and a notch feature;
FIG. 14B depicts a perspective view of the staple of FIG. 14A, but shown in the formed state;
FIG. 15 depicts a perspective view of an exemplary staple having a "V" shape and reduced diameter feature;
FIG. 16 depicts a front view of an exemplary "V" shaped staple having a flat area feature;
FIG. 17 depicts a front view of an exemplary "V" shaped staple having a hinge feature.
FIG. 18 depicts a front view of an exemplary "V" shaped staple having an alternate hinge feature.
FIG. 19 depicts a front view of an exemplary "V" shaped staple having a cable connecting portions of a crown of the staple;
FIGS. 20A-20D depicts top views of exemplary staple patterns; and
FIG. 21 depicts a top view of exemplary "C" shaped staples in a two-row nested arrangement.

The drawings are not intended to be limiting in any way.

### DETAILED DESCRIPTION

The following description of certain examples of the technology should not be used to limit its scope. Accordingly, the drawings and descriptions should be regarded as illustrative in nature and not restrictive.

For clarity of disclosure, the terms "proximal" and "distal" are defined herein relative to a surgeon, or other operator, grasping a surgical instrument having a distal surgical end effector. The term "proximal" refers to the position of an element arranged closer to the surgeon, and the term "distal" refers to the position of an element arranged closer to the surgical end effector of the surgical instrument and further away from the surgeon. Moreover, to the extent that spatial terms such as "top," "bottom," "upper," "lower," "vertical," "horizontal," or the like are used herein with reference to the drawings, it will be appreciated that such terms are used for exemplary description purposes only and are not intended to be limiting or absolute. In that regard, it will be understood that surgical instruments such as those disclosed herein may be used in a variety of orientations and positions not limited to those shown and described herein.

Furthermore, the terms "about," "approximately," and the like as used herein in connection with any numerical values or ranges of values are intended to encompass the exact value(s) referenced as well as a suitable tolerance that enables the referenced feature or combination of features to function for the intended purpose described herein.

### I. Overview of Exemplary Circular Surgical Stapling Instrument

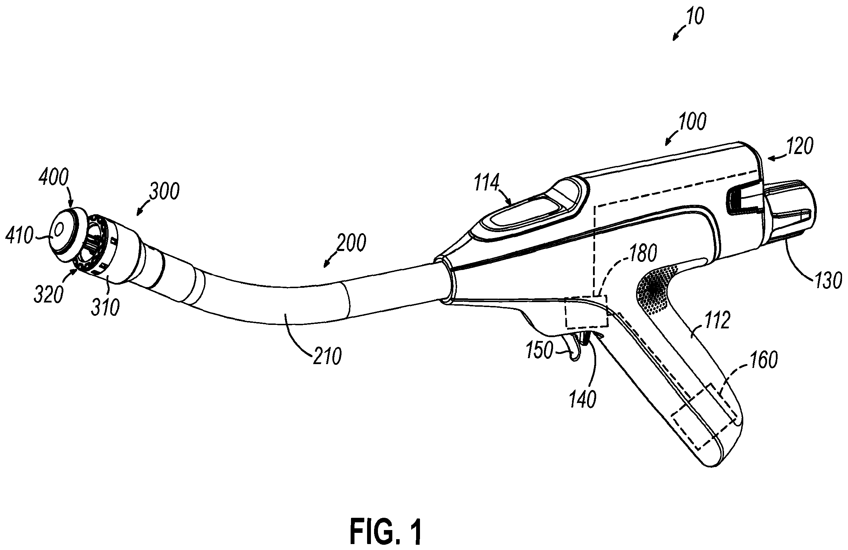

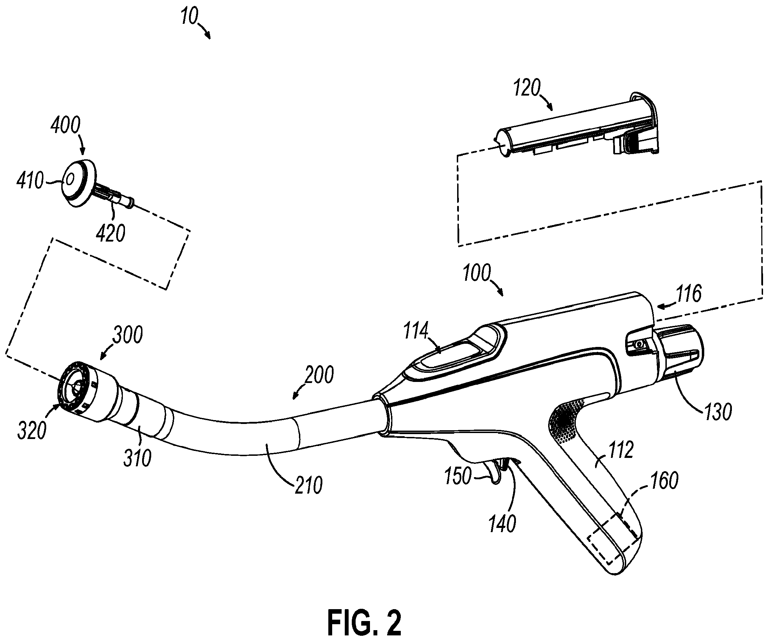

FIGS. 1-2 depict an exemplary circular surgical stapling instrument (10) that may be used to provide an end-to-end, side-to-side, or end-to-side anastomosis between two sections of an anatomical lumen such as a portion of a patient's digestive tract. Instrument (10) of this example includes a body assembly in the form of a handle assembly (100), a shaft assembly (200) extending distally from handle assembly (100), a stapling head assembly (300) at a distal end of shaft assembly (200), and an anvil (400) configured to releasably couple and cooperate with stapling head assembly (300) to clamp, staple, and cut tissue. Instrument (10) further includes a removable battery pack (120) operable to provide electrical power to a motor (160) housed within handle assembly (100), as will be described in greater detail below.

As shown in FIGS. 1-2 and as will be described in greater detail below, anvil (400) is configured to removably couple with shaft assembly (200), adjacent to stapling head assembly (300). As will also be described in greater detail below, anvil (400) and stapling head assembly (300) are configured to cooperate to manipulate tissue in three ways, including clamping the tissue, cutting the tissue, and stapling the tissue. A rotatable knob (130) at the proximal end of handle assembly (100) is rotatable to provide precise clamping of the tissue between anvil (400) and stapling head assembly (300). When a safety trigger (140) of handle assembly (100) is pivoted away from a firing trigger (150) of handle assembly (100), firing trigger (150) may be actuated to thereby provide cutting and stapling of the clamped tissue.

### A. Exemplary Anvil

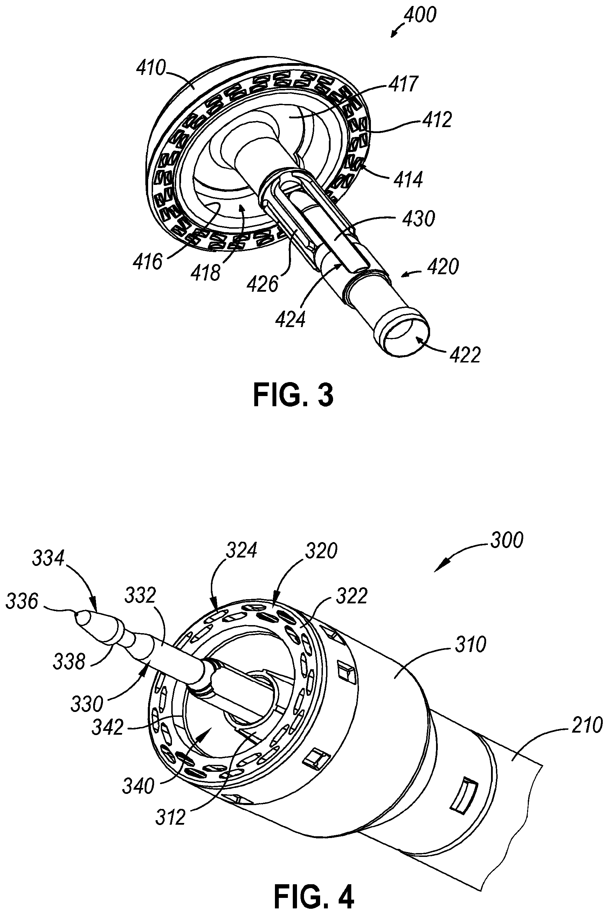

As best seen in FIG. 3, anvil (400) of the present example comprises a head (410) and a shank (420). Head (410) includes a proximal stapling surface (412) that defines a plurality of staple forming pockets (414). Staple forming pockets (414) are arranged in two concentric annular arrays in the present example. Staple forming pockets (414) are configured to deform staples as the staples are driven into staple forming pockets (414). Proximal stapling surface (412) terminates at an inner edge (416), which defines an outer boundary of an annular recess (418) surrounding shank (420). A breakable washer (417) is positioned within annular recess (418) and is configured to provide the operator with a tactile and audible indication that a distal firing stroke has been completed, in addition to serving as a cutting board, as described in greater detail below.

Shank (420) defines a bore (422) and includes a pair of pivoting latch members (430). Latch members (430) are positioned within bore (422) such that distal ends (434) are positioned at the proximal ends of lateral openings (424), which are formed through the sidewall of shank (420). Latch members (430) thus act as retaining clips. This allows anvil (400) to be removably secured to an actuatable closure member in the form of a trocar (330) of stapling head assembly (300), as will be described in greater detail below. Shank (420) of anvil (400) and trocar (330) of stapling head assembly (300) thus cooperate with one another as coupling members.

### B. Exemplary Stapling Head Assembly

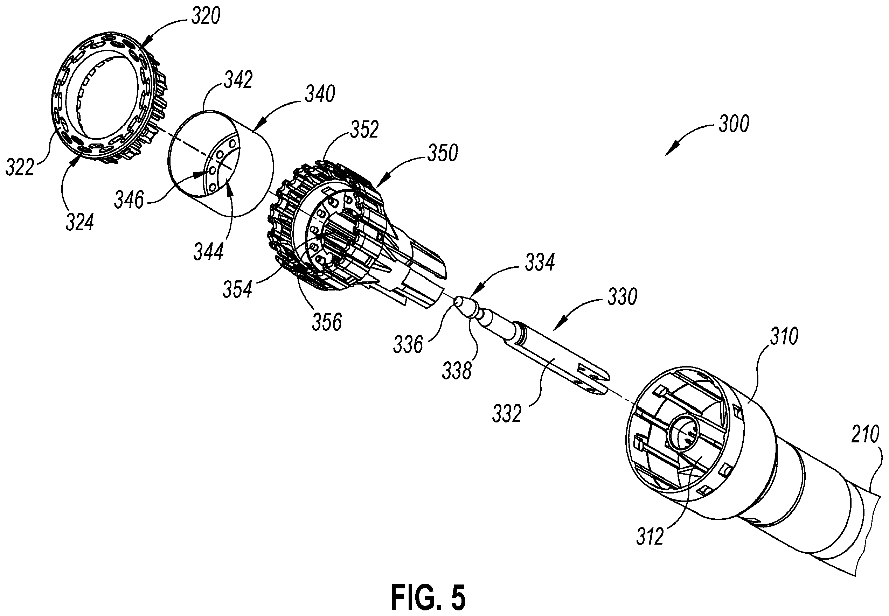

As best seen in FIGS. 4 and 5, stapling head assembly (300) of the present example is coupled to a distal end of shaft assembly (200) and comprises a tubular body member (310) and a staple driver member (350) slidably housed therein. Body member (310) includes a distally extending cylindraceous inner core member (312) positioned coaxially therein. Body member (310) is fixedly secured to an outer sheath (210) of shaft assembly (200), and body member (310) and outer sheath (210) thus serve together as a mechanical ground for stapling head assembly (300).

Trocar (330) is positioned coaxially within inner core member (312) of body member (310). As will be described in greater detail below, trocar (330) is operable to translate distally and proximally relative to body member (310) in response to rotation of knob (130) relative to casing (110) of handle assembly (100). Trocar (330) comprises a shaft (332) and a head (334). Head (334) includes a pointed tip (336) and a radially inwardly extending proximal surface (338). Head (334) and the distal portion of shaft (332) are configured for insertion into bore (422) of anvil (400). Proximal surface (338) and latch shelves (436) have complementary positions and configurations such that latch shelves (436) engage proximal surface (338) when shank (420) of anvil (400) is fully seated on trocar (330). Anvil (400) is thus secured to trocar (330) through a snap fit provided by latch members (430).

Staple driver member (350) is operable to actuate longitudinally within body member (310) in response to activation of motor (160) as will be described in greater detail below. As shown best in FIG. 5, staple driver member (350) of the present example includes two distally presented concentric annular arrays of staple drivers (352). Staple drivers (352) are arranged to correspond with the arrangement of staple forming pockets (414) of anvil (400). Thus, each staple driver (352) is configured to drive a corresponding staple distally into a corresponding staple forming pocket (414) when stapling head assembly (300) is actuated (or "fired"). Staple driver member (350) also defines a bore (354) that is configured to coaxially and slidably receive core member (312) of body member (310). An annular array of studs (356) project distally from a distally presented surface surrounding bore (354).

A cylindraceous knife member (340) is coaxially positioned within a distally-opening central recess of staple driver member (350) that communicates with bore (354). Knife member (340) includes a distally presented, sharp circular cutting edge (342). Knife member (340) is sized such that knife member (340) defines an outer diameter that is just smaller than the diameter defined by the radially inner-most surfaces of the inner annular array of staple drivers (352). Knife member (340) also defines a central opening that is configured to coaxially receive core member (312) of body member (310). An annular array of openings (346) formed in knife member (340) is configured to mate with the annular array of studs (356) of staple driver member (350), such that knife member (340) is fixedly secured to staple driver member (350) via studs (356) and openings (346).

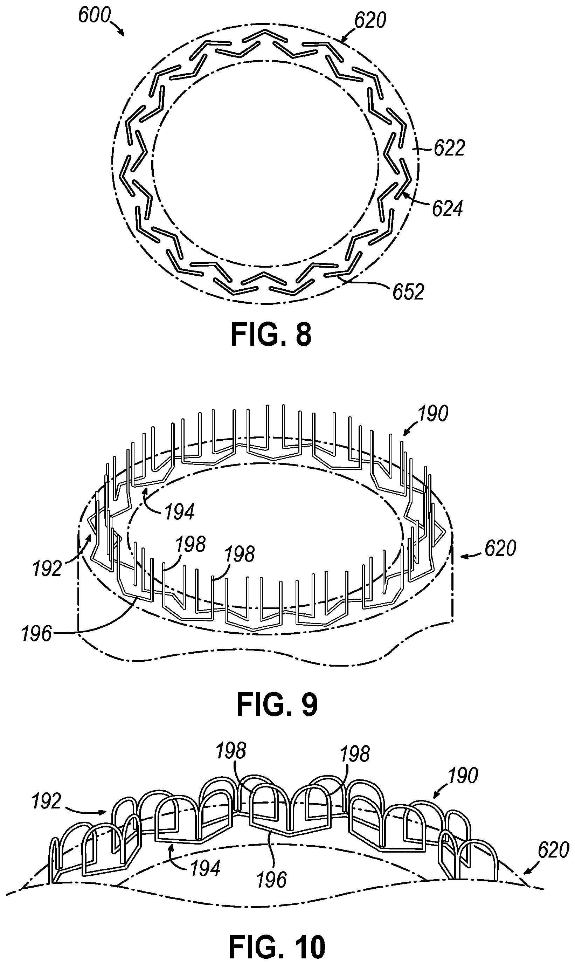

An annular deck member (320) is fixedly secured to a distal end of body member (310). Deck member (320) includes a distally presented stapling surface in the form of a deck surface (322) having two concentric annular arrays of staple openings (324). Staple openings (324) are arranged to align with the arrangement of staple drivers (352) of staple driver member (350) and staple forming pockets (414) of anvil (400) described above. Each staple opening (324) is configured to slidably receive and provide a pathway for a corresponding staple driver (352) to drive a corresponding staple distally through deck member (320) and into a corresponding staple forming pocket (414) when stapling head assembly (300) is actuated. As best seen in FIG. 9, deck member (320) has a central opening that defines an inner diameter that is just slightly larger than the outer diameter defined by knife member (340). Deck member (320) is thus configured to permit knife member (340) to translate longitudinally through the central opening concurrently with longitudinal translation of staple driver member (350). In particular, knife member (340) is configured to actuate relative to deck member (340) between a proximal retracted position and a distal extended position, where cutting edge (342) is proximal to deck surface (322) in the proximal retracted position and distal to deck surface (322) in the distal extended position.

### C. Exemplary Shaft Assembly

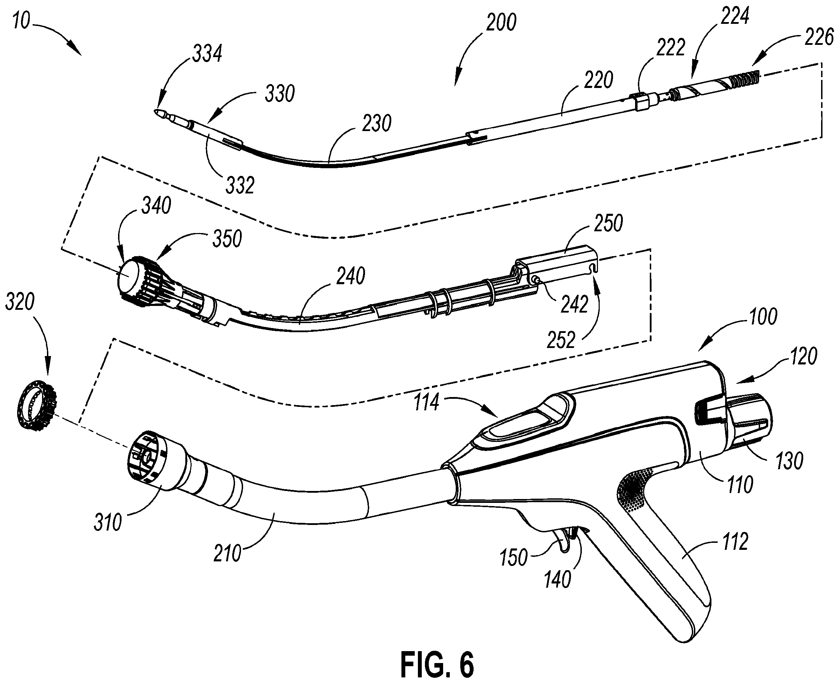

FIG. 6 shows various components of shaft assembly (200), which operatively couple components of stapling head assembly (300) with components of handle assembly (100). In particular, and as noted above, shaft assembly (200) includes an outer sheath (210) that extends between handle assembly (100) and body member (310) and includes a medial portion that extends along a curved path.

Shaft assembly (200) further includes a trocar actuation rod (220) having a proximal end operatively coupled with rotatable knob (130) and a distal end coupled with a flexible trocar actuation band assembly (230), the assembly of which is slidably housed within outer sheath (210). The distal end of trocar actuation band assembly (230) is fixedly secured to the proximal end of trocar shaft (332), such that trocar (330) will translate longitudinally relative to outer sheath (210) in response to translation of trocar actuation band assembly (230) and trocar actuation rod (220) relative to outer sheath (210), which occurs in response to rotation of rotatable knob (130). A clip (222) is fixedly secured to trocar actuation rod (220) and is configured to cooperate with complementary features within handle assembly (100) to prevent trocar actuation rod (220) from rotating within handle assembly (100) while still permitting trocar actuation rod (220) to translate longitudinally within handle assembly (100). Trocar actuation rod (220) further includes a section of coarse helical threading (224) and a section of fine helical threading (226) proximal to coarse helical threading (224), which are configured to control a rate of longitudinal advancement of trocar actuation rod (220), as described in greater detail below.

Shaft assembly (200) further includes a stapling head assembly driver (240) that is slidably housed within outer sheath (210) and about the combination of trocar actuation rod (220) and trocar actuation band assembly (230). Stapling head assembly driver (240) includes a distal end that is fixedly secured to the proximal end of staple driver member (350), a proximal end secured to a drive bracket (250) via a pin (242), and a flexible section disposed therebetween. It should therefore be understood that staple driver member (350) will translate longitudinally relative to outer sheath (210) in response to translation of stapling head assembly driver (240) and drive bracket (250) relative to outer sheath (210).

### D. Exemplary Handle Assembly and User Input Features

As shown in FIG. 1, handle assembly (100) includes a casing (110) having a lower portion that defines an obliquely oriented pistol grip (112) and an upper portion that supports a user interface feature (114) and releasably receives a battery pack (120), as described in greater detail below. Handle assembly (100) further includes several features that are operable to actuate anvil (400) and stapling head assembly (300). In particular, handle assembly (100) includes a rotatable knob (130), a safety trigger (140), a firing trigger (150), a motor (160), and a motor activation module (180). Knob (130) is coupled with trocar actuation rod (220) via a nut (not shown), such that coarse helical threading (224) will selectively engage a thread engagement feature within the interior of the nut; and such that fine helical threading (226) will selectively engage a thread engagement feature within the interior of knob (130). These complementary structures are configured such that trocar actuation rod (220) will first translate proximally at a relatively slow rate, and then translate proximally at a relatively fast rate, in response to rotation of knob (130).

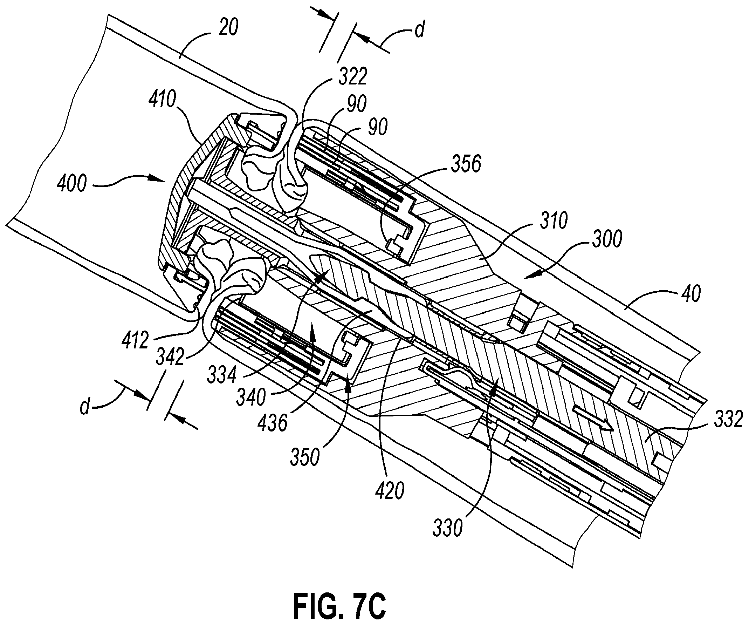

It should be understood that when anvil (400) is coupled with trocar (330), rotation of knob (130) will provide corresponding translation of anvil (400) relative to stapling head assembly (300). It should also be understood that knob (130) may be rotated in a first angular direction (e.g., clockwise) to retract anvil (400) proximally toward stapling head assembly (300); and in a second angular direction (e.g., counterclockwise) to extend anvil (400) distally away from stapling head assembly (300). Knob (130) may thus be used to adjust a gap distance (d) between opposing stapling surfaces (412, 322) of anvil (400) and stapling head assembly (300) until a suitable gap distance (d) has been achieved, for example as shown in FIG. 7C described below.

Firing trigger (150) is operable to activate motor (160) to thereby actuate stapling head assembly (300) to staple and cut tissue clamped between anvil (400) and stapling head assembly (300). Safety trigger (140) is operable to selectively block actuation of firing trigger (150) based on the longitudinal position of anvil (400) in relation to stapling head assembly (300). Handle assembly (100) also includes components that are operable to selectively lock out both triggers (140, 150) based on the position of anvil (400) relative to stapling head assembly (300). For instance, safety trigger (140) may be blocked from rotating from an engaged position to a disengaged position until the position of anvil (400) relative to stapling head assembly (300) is within a predefined range. Accordingly, until the anvil position is within the predefined range, actuation of firing trigger (150) is blocked by safety trigger (140), thereby inhibiting firing of stapling head assembly (300).

Firing trigger (150) is operable to actuate a switch of motor activation module (180) (FIG. 1) when firing trigger (150) is pivoted proximally to a fired position. Motor activation module (180) is in communication with battery pack (120) and motor (160), such that motor activation module (180) is configured to provide activation of motor (160) with electrical power from battery pack (120) in response to firing trigger (150) actuating the switch of motor activation module (180). Thus, motor (160) will be activated when firing trigger (150) is pivoted. This activation of motor (160) will actuate stapling head assembly (300) via drive bracket (250), as described in greater detail below.

### E. Exemplary Anastomosis Procedure with Circular Stapling Instrument

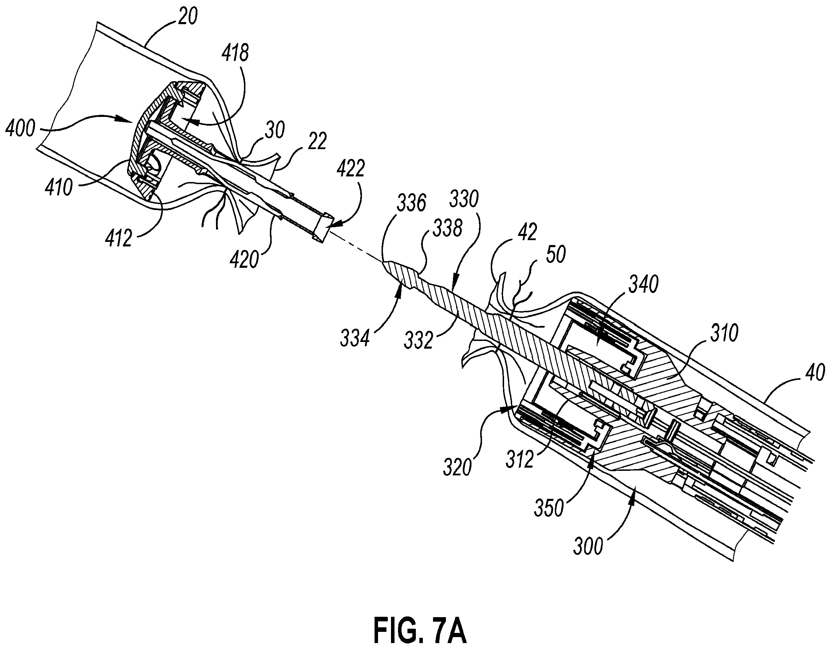

FIGS. 7A-7E show instrument (10) being used to form an anastomosis (70) between two tubular anatomical structures (20, 40). By way of example only, the tubular anatomical structures (20, 40) may comprise sections of a patient's esophagus, colon, or other portions of the patient's digestive tract, or any other tubular anatomical structures.

As shown in FIG. 7A, anvil (400) is positioned in one tubular anatomical structure (20) and stapling head assembly (300) is positioned in another tubular anatomical structure (40). As shown in FIG. 7A, anvil (400) is positioned in tubular anatomical structure (20) such that shank (420) protrudes from the open severed end (22) of tubular anatomical structure (20). In the present example, purse-string suture (30) is provided about a mid-region of shank (420) to generally secure the position of anvil (400) in tubular anatomical structure (20). Stapling head assembly (300) is positioned in tubular anatomical structure (40) such that trocar (330) protrudes from the open severed end (42) of tubular anatomical structure (20). A purse-string suture (50) is provided about a mid-region of shaft (332) to generally secure the position of stapling head assembly (300) in tubular anatomical structure (40). Stapling head assembly (300) is then urged distally to ensure that stapling head assembly (300) is fully seated at the distal end of tubular anatomical structure (40).

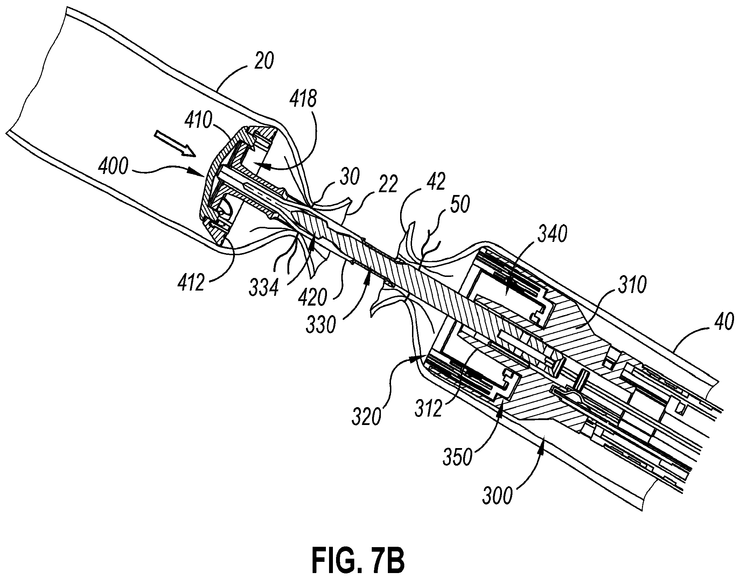

Next, anvil (400) is secured to trocar (330) by inserting trocar (330) into bore (422) as shown in FIG. 7B. Latch members (430) of anvil (400) engage head (334) of trocar (330), thereby providing a secure fit between anvil (400) and trocar (330). The operator then rotates knob (130) while holding casing (110) stationary via pistol grip (112). This rotation of knob (130) causes trocar (330) and anvil (400) to retract proximally. As shown in FIG. 7C, this proximal retraction of trocar (330) and anvil (400) compresses the tissue of tubular anatomical structures (20, 40) between surfaces (412, 322) of anvil (400) and stapling head assembly (300). As this occurs, the operator may observe the tactile resistance or feedback via knob (130) while turning knob (130), with such tactile resistance or feedback indicating that the tissue is being compressed. As the tissue is being compressed, the operator may visually observe the position of an indicator needle (not shown) within user interface feature (114) of handle assembly (100) to determine whether the gap distance (d) between opposing surfaces (412, 322) of anvil (400) and stapling head assembly (300) is appropriate; and make any necessary adjustments via knob (130).

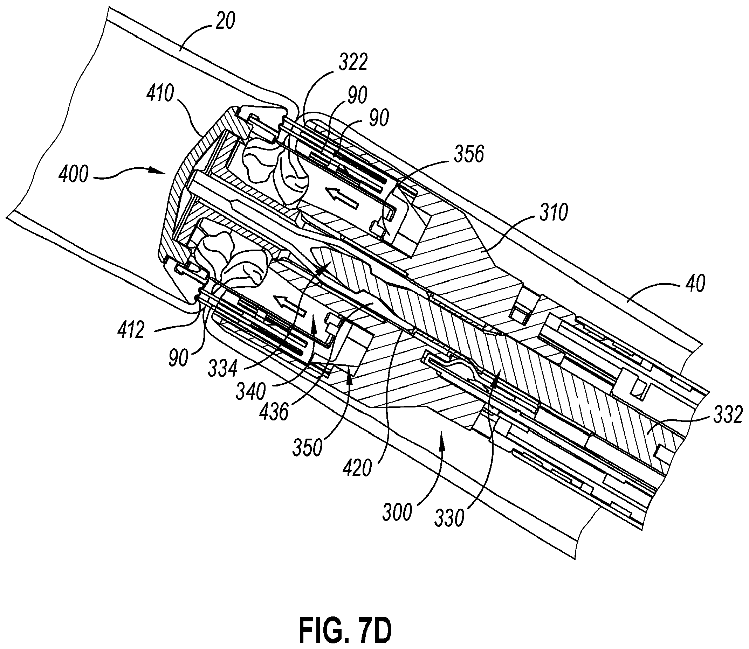

Once the operator has appropriately set the gap distance (d) via knob (130), the operator pivots safety trigger (140) toward pistol grip (112) to enable actuation of firing trigger (150). The operator then pivots firing trigger (150) toward pistol grip (112), thus causing firing trigger (150) to actuate the switch of motor activation module (180) and thereby activate motor (160) to rotate. This rotation of motor (160) causes actuation (or "firing") of stapling head assembly (300) by actuating drive bracket (250) distally to thereby drive knife member (340) and staple driver member (350) distally together, as shown in FIG. 7D.

As knife member (340) translates distally, cutting edge (342) of knife member (340) cuts excess tissue that is positioned within annular recess (418) of anvil (400) and the interior of knife member (340). Additionally, washer (417) positioned within annular recess (418) of anvil (400) is broken by knife member (340) when the knife member (340) completes a full distal range of motion from the position shown in FIG. 7C to the position shown in FIG. 7D. It should be understood that washer (417) may also serve as a cutting board for knife member (340) to assist in cutting of tissue.

As staple driver member (350) translates distally from the position shown in FIG. 7C to the position shown in FIG. 7D, staple driver member (350) drives staples (90) through the tissue of tubular anatomical structures (20, 40) and into staple forming pockets (414) of anvil (400). Staple forming pockets (414) deform the driven staples (90) into a "B" shape or a three-dimensional shape, for example, such that the formed staples (90) secure the ends of tissue together, thereby coupling tubular anatomical structure (20) with tubular anatomical structure (40).

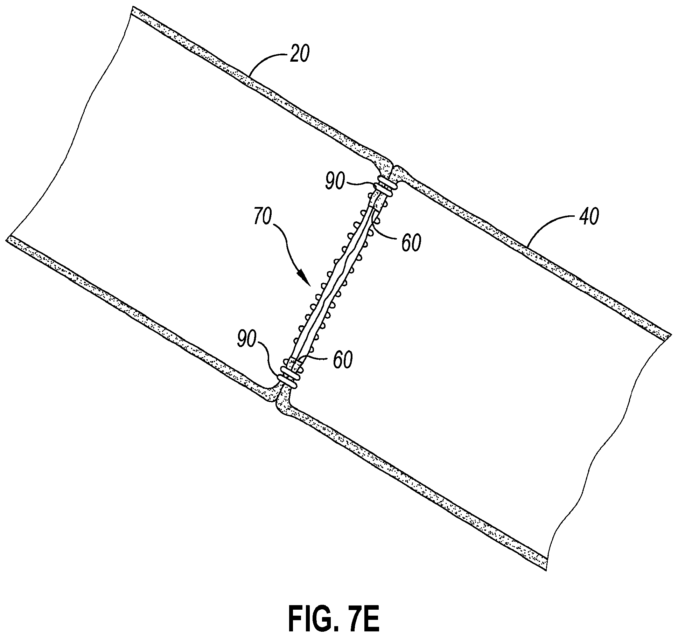

After the operator has actuated (or "fired") stapling head assembly (300) as shown in FIG. 7D, the operator rotates knob (130) to drive anvil (400) distally away from stapling head assembly (300), thereby increasing the gap distance (d) to facilitate release of the tissue between surfaces (412, 322). The operator then removes instrument (10) from the patient, with anvil (400) still secured to trocar (330). With instrument (10) removed, the tubular anatomical structures (20, 40) are left secured together by two annular arrays of staples (90) at an anastomosis (70) as shown in FIG. 7E. The inner diameter of the anastomosis (70) is defined by the severed edge (60) left by knife member (340).

### II. Exemplary Stapling Head Assembly with Staples Having Expandable Crowns

As noted above, the inner diameter of anastomosis (70) formed by instrument (10) is defined by the outer diameter of knife member (340). Because knife member (340) is smaller than the inner diameters of tubular anatomical structures (20, 40), the resulting diameter of anastomosis (70) is generally smaller than that of each tubular anatomical structure (20, 40). Additionally, the configuration of formed staples (90) may inhibit the ability of anastomosis (70) to expand radially.

In some procedures, it may be desirable to form an anastomosis (70) of enlarged diameter and/or to enable the annular arrays of formed staples (90) to expand radially, thereby minimizing strictures, enabling better peristalsis, and minimizing local tension in and resulting damage to the joined portions of tubular anatomical structures (20, 40). Accordingly, in some such instances, it may be desirable to configure stapling head assembly (300) and anvil (400) with features that enable formation of such an anastomosis and/or patterns of formed staples (90). Exemplary versions of such features are described in greater detail below.

### A. Exemplary Stapling Head Assembly with "V" Shaped Expandable Staples

FIGS. 8-12 illustrate portions of an exemplary stapling head assembly (600) that is similar to exemplary stapling head assembly (300) described above. In some versions, stapling instrument (10) can be fitted with stapling head assembly (600) instead of stapling head assembly (300) and be operable as described above. Stapling head assembly (600) is configured the same as stapling head assembly (300) except stapling head assembly (600) includes deck member (620) with deck surface (622) and staple openings (624). Also, staple driver member (350), when used with stapling head assembly (600) is configured with staple drivers (652) that have a differing shape from staple drivers (352) as will be discussed further below. Furthermore, stapling head assembly (600) is configured for use with expandable staples generally having a "V" shape as will be described further below.

### 1. Exemplary Expandable Staples with "V" Shaped Crown

Referring to FIGS. 8-12, stapling head assembly (600) is configured for use with expandable staples (190). In the present example, staples (190) are configured with a "V" shape that allows each staple (190) to expand after being deployed as will be discussed in greater detail below. Referring to FIG. 8, deck member (620) is shown with deck surface (622) having "V" shaped openings (624). With staples (190) not shown in FIG. 8, staples drivers (652) are visible through openings (624). As shown, staple drivers (652) have a corresponding "V" shape to openings (624) such that staple drivers (652) are configured to deploy staples (190), with their "V" shape, through openings (624).

Referring to FIG. 9, a portion of deck member (620) is shown in phantom to reveal a plurality of undeployed staples (190) loaded within deck member (620). As shown in the present example, staples (190) are arranged in two annular or ring-shaped rows where there is an outer row (192) and an inner row (194). Staples (190) are further arranged such that each row is staggered or offset from the other, and such that staples (190) in one row oppose staples (190) in the other row. With this opposing arrangement, staples (190) in one row are effectively rotated 180 degrees from staples (190) in the other row. In some versions, the size and/or shape of staples (190) in inner row (194) can differ from that of outer row (192) to assist in establishing the circular staple pattern shown.

Staples (190) each comprise a crown (196) and a pair of legs (198). Furthermore, staples (190) have a "V" shape where a top view looking down onto crown (196) presents a "V" shape. The "V" shape can further be described as having two straight portions or lines with a vertex between them. In this fashion, staples (190) define an angle between the pair of legs (198). As understood from comparing FIGS. 9 and 10, staples (190) have this "V" shape in both the undeployed state as shown in FIG. 9 and in the deployed state as shown in FIG. 10. As shown in FIG. 10, when staples (190) are deployed or fired from instrument (10), as described above, the interaction of staples (190) with anvil (400) deforms the pair of legs (198) to bend them in a curved manner forming a "B" shape.

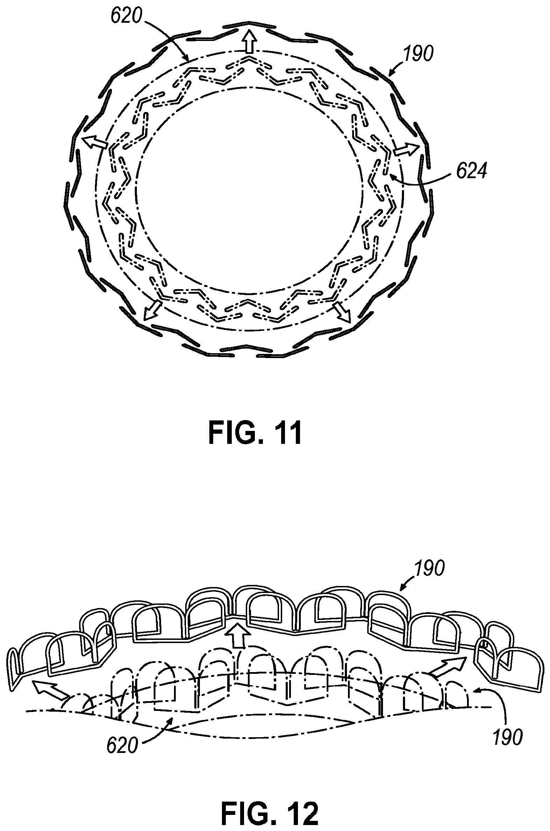

Referring now to FIGS. 11 and 12, staples (190) are configured as expandable staples. For instance, FIG. 11 illustrates the staple deck (620) showing openings (624) from which staples (190) would have initially been deployed. As shown in FIG. 11 and further in FIG. 12, after staples (190) have been deployed they may undergo an expansion allowing them to move radially. This expansion allows for the plurality of staples (190) forming the ring shape or pattern to define a first or initial diameter, and then subsequently allowing the same plurality of staples (190) to define a second or subsequent diameter that is larger than the initial diameter. In this manner, the diameter of the lumen at the anastomosis is increased.

In the present example, the "V" shape configuration of staples (190) contributes to the ability of staples (190) to expand or be expandable. In some versions, staples (190) are formed with the "V" shape and after deployment, staples (190) can experience tension based on radial tissue forces, e.g., from peristalsis, or from the passage of material through the lumen. In response to experiencing this tension, legs (198) of staples (190) deflect from the vertex to define a larger angle between the pair of legs (198). Consequently, the width of staples (190) increase, with the width being defined as the linear distance between the pair of legs (198).

In other versions, staples (190) initially have a straight or substantially straight shape and are bent when loaded within instrument (10). For instance, staples (190) may be bent into a "V" shape when loaded within annular deck member (320). In this manner, staples (190) are under tension when deployed, but then after deployment, staples (190) return to their relaxed state and thus expand radially by expanding to a straighter shape. In this version, staples (190) automatically expand after being deployed without needing any tissue force placed upon staples (190) to achieve expansion of staples (190). As will be described further below, other "V" shape staples having other features that promote expandability can be used with instrument (10) and stapling head assembly (600) in place of staples (190).

### 2. Exemplary Expandable Staples with "V" Shaped Crown and Spring Feature

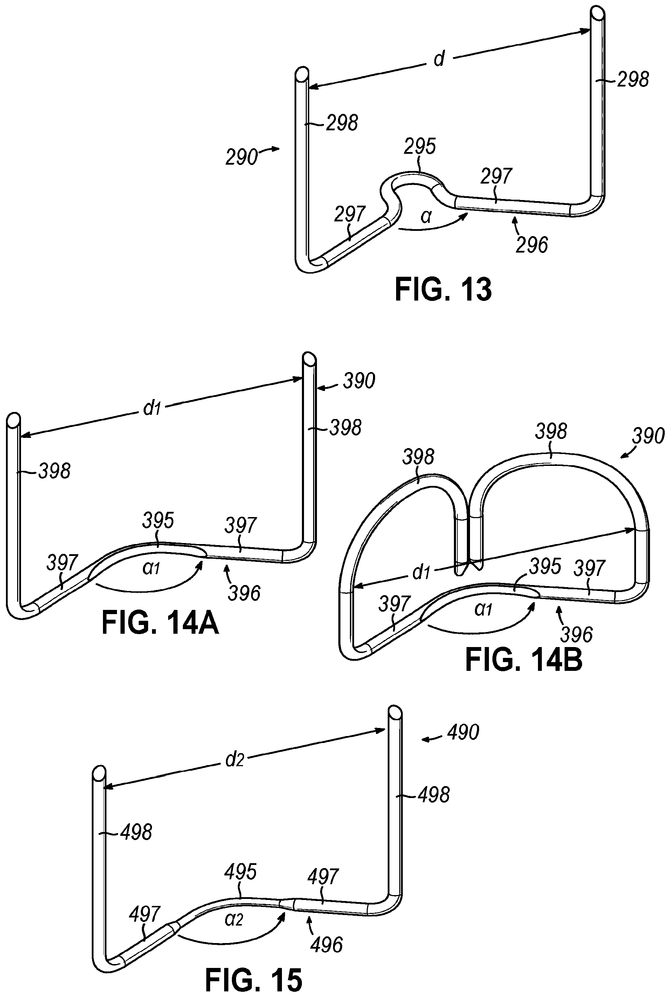

FIG. 13 depicts an exemplary staple (290), which can replace staple (190) or (90) in the plurality of staples. Staple (290) includes crown (296) and a pair of legs (298). Crown (296) includes two straight portions (297) and a spring feature (295). Spring feature (295) in the present example is located between each of straight portions (297) and is formed with a curved shape. In the unexpanded state as shown in FIG. 13, staple (290) defines an angle (α) between straight portions (297), and staple (290) defines a distance (d) between legs (298). After deployment and after staple (290) expands the same or similar to the expansion described above with respect to staple (190), staple (290) expands about spring feature (295) such that angle (α) increases, which also corresponds with an increase in distance (d). This produces a broader "V" shape for staples (290) that resembles staples (290) becoming closer to linear shaped.

In the present example, staple (290) starts initially as straight or substantially straight and adopts its "V" shape with incorporated spring feature (295) during loading with instrument (10) such that staples (290) are held within instrument (10) under tension. Moreover, staples (290) are formed of fully or partially resilient material such that after being deployed, spring feature (295) assists in automatically expanding staples (290) as they return to their relaxed state. During this expansion, staples (290) increase in width (d) between legs (298), which widens the diameter of the anastomosis.

In some other versions, staple (290) is formed such that in its relaxed state staple (290) has the "V" shape with spring feature (295). In these versions, spring feature (295) promotes expansion of staple (290) based on tissue forces described above acting on staple (290). For instance, spring feature (295) is coplanar with crown (296) and legs (298) extend generally orthogonal to a plane defined by crown (296) and spring feature (295). After deployment of staple (290), tension from radial tissue forces act on staple (290) and such forces are generally orthogonal to axes defined by legs (298) when legs (298) are in the unformed state. In this manner, spring feature (295) is configured and oriented so that when radial tissue forces act on staple (290), expansion of staple (290) occurs as described above. In view of the teachings herein, other ways to configure staple (290) with spring feature (295) will be apparent to those of ordinary skill in the art.

### 3. Exemplary Expandable Staples with "V" Shaped Crown and Weakened Region

FIGS. 14A and 14B illustrate another exemplary staple (390) which can replace staple (190) or (90) in the plurality of staples. Staple (390) includes crown (396) and a pair of legs (398). Crown (396) includes two straight portions (397) and a weakened region (395). Weakened region (395) in the present example is located between each of straight portions (397) and is formed as a notch or cut-away portion of crown (396). This configuration for weakened region (395) provides for a thinner portion of crown (396) at the junction or vertex of straight portions (397) where there is less material present. This in turn allows for staple (390) to be more responsive to expand when lower tissue forces act on staple (390).

In the undeployed state as shown in FIG. 14A, staple (390) defines an angle (α1) between straight portions (397), and staple (390) defines a distance (d1) between legs (398). After deployment as shown in FIG. 14B, and after staple (390) is subject to tension from tissue forces as described above, staple (390) expands about weakened region (395) such that angle (α1) increases, which also corresponds with an increase in distance (d1). This produces a broader "V" shape for staples (390) that resembles staples (390) becoming closer to linear shaped. In the present example, staple (390) with weakened region (395) is configured such that it plastically deforms when the tissue forces act on staple (390) as described above. In this manner, the increased diameter at the anastomosis can be maintained.

As shown in FIGS. 14A and 14B, weakened region (395) is coplanar with crown (396). Additionally, legs (398) extend generally orthogonal to a plane defined by crown (396) and weakened region (395). After deployment of staples (390), tension from radial tissue forces act on staple (390) and such forces are generally orthogonal to axes defined by legs (398) when in the unformed state. In this manner, weakened region (395) is configured and oriented so that when radial tissue forces act on staple (390), expansion of staple (390) occurs as described above. In view of the teachings herein, other ways to configure staple (390) with weakened region (395) will be apparent to those of ordinary skill in the art.

FIG. 15 illustrates another exemplary staple (490) which can replace staple (190) or (90) in the plurality of staples. Staple (490) includes crown (496) and a pair of legs (498). Crown (496) includes two straight portions (497) and a weakened region (495). Weakened region (495) in the present example is located between each of straight portions (497) and is formed as a reduced diameter section of crown (496). This configuration for weakened region (495) provides for a smaller portion of crown (496) at the junction or vertex of straight portions (497) where there is less material present. This in turn allows for staple (490) to be more responsive to expand when lower tissue forces act on staple (490).

In the undeployed state as shown in FIG. 15, staple (490) defines an angle (α2) between straight portions (497), and staple (490) defines a distance (d2) between legs (498). After deployment, and after staple (490) is subject to tension from tissue forces as described above, staple (490) expands about weakened region (495) such that angle (α2) increases, which also corresponds with an increase in distance (d2). This produces a broader "V" shape for staples (490) that resembles staples (490) becoming closer to linear shaped. In the present example, staple (490) with weakened region (495) is configured such that it plastically deforms when the tissue forces act on staple (490) as described above. In this manner, the increased diameter at the anastomosis can be maintained.

As shown in FIG. 15, weakened region (495) is coplanar with crown (496). Additionally, legs (498) extend generally orthogonal to a plane defined by crown (496) and spring feature (495). After deployment of staples (490), tension from radial tissue forces act on staple (490) and such forces are generally orthogonal to axes defined by legs (497) when in the unformed state. In this manner, weakened region (495) is configured and oriented so that when radial tissue forces act on staple (490), expansion of staple (490) occurs as described above. In view of the teachings herein, other ways to configure staple (490) with weakened region (495) will be apparent to those of ordinary skill in the art.

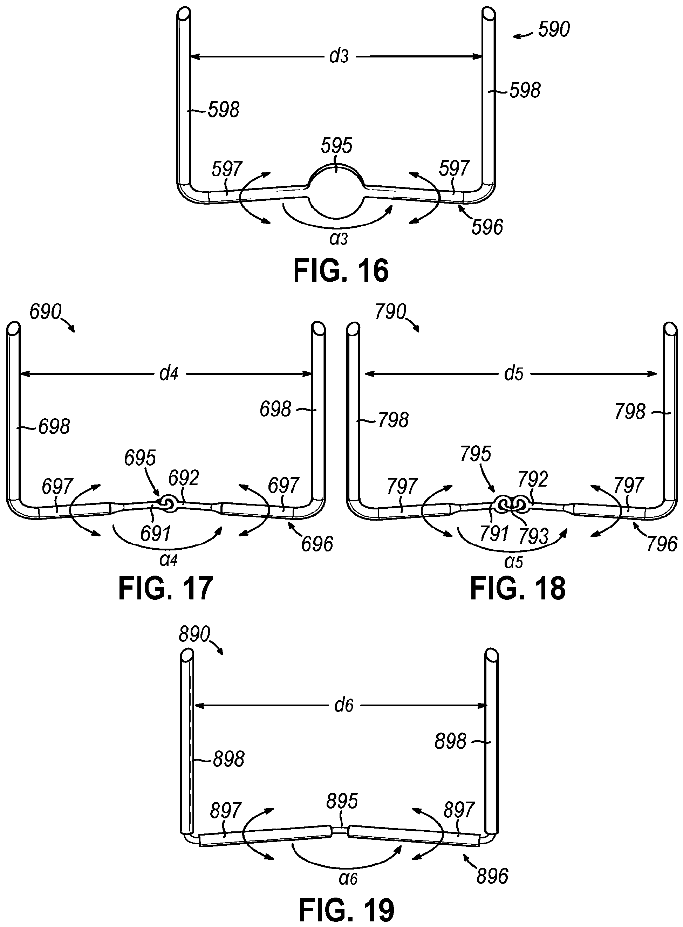

FIG. 16 illustrates another exemplary staple (590) which can replace staple (190) or (90) in the plurality of staples. Staple (590) includes crown (596) and a pair of legs (598). Crown (596) includes two straight portions (597) and a weakened region (595). Weakened region (595) in the present example is located between each of straight portions (597) and is formed as a flat area or portion of crown (596). This configuration for weakened region (595) provides for a more pliable portion of crown (596) at the junction or vertex of straight portions (597) where there is thinner material present. This in turn allows for staple (590) to be more responsive to expand when lower tissue forces act on staple (590).

In the undeployed state as shown in FIG. 16, staple (590) defines an angle (α3) between straight portions (597), and staple (590) defines a distance (d3) between legs (598). After deployment, and after staple (590) is subject to tension from tissue forces as described above, staple (590) expands about weakened region (595) such that angle (α3) increases, which also corresponds with an increase in distance (d3). This produces a broader "V" shape for staples (590) that resembles staples (590) becoming closer to linear shaped. In the present example, staple (590) with weakened region (595) is configured such that it plastically deforms when the tissue forces act on staple (590) as described above. In this manner, the increased diameter at the anastomosis can be maintained.

As shown in FIG. 16, legs (598) extend generally orthogonal to a plane defined by crown (596). Similarly, weakened region (595), and its flat area, extend generally orthogonal to the plane defined by crown (596). In use after deployment of staples (590), tension from radial tissue forces act on staple (590) and such forces are generally orthogonal to axes defined by legs (598) when in the unformed state. Accordingly, in this manner, weakened region (595) is configured and oriented so that when radial tissue forces act on staple (590), expansion of staple (590) occurs as described above. In view of the teachings herein, other ways to configure staple (590) with spring feature (595) will be apparent to those of ordinary skill in the art.

Staples (390, 490, 590) above are described as being pre-formed with a "V" shape and a weakened region (395, 495, 595) where these staples maintain their "V" shape in a relaxed state and expand when subjected to tissue forces. However, in some other versions, staples (390, 490, 590) can be configured similar to staple (290), where staples (390, 490, 590) are initially straight or substantially straight and when loaded within instrument (10), staples (390, 490, 590) are bent to the "V" shape and held under tension. After deployment, staples (390, 490, 590) automatically expand to their relaxed straighter configuration.

### 4. Exemplary Expandable Staples with "V" Shaped Crown and Hinge Feature

FIG. 17 depicts an exemplary staple (690), which can replace staple (190) or (90) in the plurality of staples. Staple (690) includes crown (696) and a pair of legs (698). Crown (696) includes two straight portions (697) and a hinge feature (695). Hinge feature (695) in the present example is located between each of straight portions (697) and is formed as two interlocking rings or loops (691, 692). In the undeployed state as shown in FIG. 17, staple (690) defines an angle (α4) between straight portions (697), and staple (690) defines a distance (d4) between legs (698). After deployment and after staple (690) is subject to tension from tissue forces as described above, staple (690) expands about hinge feature (695) such that angle (α4) increases, which also corresponds with an increase in distance (d4). This produces a broader "V" shape for staples (690) that resembles staples (690) becoming closer to linear shaped.

In the present example, hinge feature (695) is configured such that it is operable to change the orientation of staple (690) depending on the forces staple (690) is subjected to. Also in the present example, hinge feature (695) is configurable such that a tension within hinge feature (695) can be such that a threshold amount of force must be imparted upon hinge feature (695) to cause staple (690) to change configuration as described above. For instance, in one version the amount of contact between interlocking rings (691, 692) can be configured to provide greater or lesser friction among these components, thereby making hinge feature (695) more or less responsive to a given force applied on staple (690). Other ways to configure the tension with hinge feature (695) will be apparent to those of ordinary skill in the art in view of the teachings herein.

Staple (690) can be considered to define a first portion that includes one leg (698) and one portion (697) of crown (696), and a second portion that includes the other leg (698) and the other portion (697) of crown (696). In this manner, first and second portions of staple (690) are connected at hinge feature (695) and are operable to move relative to one another about hinge feature (695) depending on the forces being exerted on the first and second portions of staple (690). In some instances, a force may be exerted evenly on staple (690) such that each of the first and second portions of staple (690) experience the same force and move relative to one another in a similar fashion. In some other instances, a different degree of force may act on each of the first and second portions of staple (690). In such examples, the degree of movement of the first and the second portions of staple (690) may differ. For example, the portion of staple (690) experiencing the larger force may move a greater distance radially compared to the other portion of staple (690) experiencing the lower force.

As shown in FIG. 17, hinge feature (695) is coplanar with crown (696). Additionally, legs (698) extend generally orthogonal to a plane defined by crown (696) and hinge feature (695). In use after deployment of staples (690), tension from radial tissue forces act on staple (690) and such forces are generally orthogonal to axes defined by legs (698) when in the unformed state. Accordingly, in this manner, hinge feature (695) is configured and oriented so that when radial tissue forces act on staple (690), expansion of staple (690) occurs as described above. While the above examples describe expansion of staple (690), in some instances, hinge feature (695) also allows for staple (690) to move or change orientation from an expanded state to a more retracted or compact state. This could be the case when forces acting on staple (690) subside. Of course, in such examples hinge feature (695) can be configured to allow for expansion only without the ability to contract or close down to a smaller width. In view of the teachings herein, other ways to configure staple (690) with hinge feature (695) will be apparent to those of ordinary skill in the art.

FIG. 18 depicts an exemplary staple (790), which can replace staple (190) or (90) in the plurality of staples. Staple (790) includes crown (796) and a pair of legs (798). Crown (796) includes two straight portions (797) and a hinge feature (795). Hinge feature (795) in the present example is located between each of straight portions (797) and is formed as two interlocking rings or loops (791, 792) with an intermediate member or link (793). In the undeployed state as shown in FIG. 18, staple (790) defines an angle (α5) between straight portions (797), and staple (790) defines a distance (d5) between legs (798). After deployment and after staple (790) is subject to tension from tissue forces as described above, staple (790) expands about hinge feature (795) such that angle (α5) increases, which also corresponds with an increase in distance (d5). This produces a broader "V" shape for staples (790) that resembles staples (790) becoming closer to linear shaped.

In the present example, hinge feature (795) is configured such that it is operable to change the orientation of staple (790) depending on the forces staple (790) is subjected to. Also in the present example, hinge feature (795) is configurable such that a tension within hinge feature (795) can be such that a threshold amount of force must be imparted upon hinge feature (795) to cause staple (790) to change configuration as described above. For instance, in one version the amount of contact between interlocking rings (791, 792) and intermediate member (793) can be configured to provide greater or lesser friction among these components, thereby making hinge feature (795) more or less responsive to a given force applied on staple (790). Other ways to configure the tension with hinge feature (795) will be apparent to those of ordinary skill in the art in view of the teachings herein.

Staple (790) can be considered to define a first portion that includes one leg (798) and one portion (797) of crown (796), and a second portion that includes the other leg (798) and the other portion (797) of crown (796). In this example each of these first and second portions connect with intermediate member (793). In this manner, first and second portions of staple (790) are connected at hinge feature (795) and are operable to move relative to one another about hinge feature (795) depending on the forces being exerted on the first and second portions of staple (790). In some instances, a force may be exerted evenly on staple (790) such that each of the first and second portions of staple (790) experience the same force and move relative to one another in a similar fashion. In some other instances, a different degree of force may act on each of the first and second portions of staple (790). In such examples, the degree of movement of the first and the second portions of staple (790) may differ. For example, the portion of staple (790) experiencing the larger force may move a greater distance radially compared to the other portion of staple (790) experiencing the lower force.

As shown in FIG. 18, hinge feature (795) is coplanar with crown (796). Additionally, legs (798) extend generally orthogonal to a plane defined by crown (796) and hinge feature (795). In use after deployment of staples (790), tension from radial tissue forces act on staple (790) and such forces are generally orthogonal to axes defined by legs (798) when in the unformed state. Accordingly, in this manner, hinge feature (795) is configured and oriented so that when radial tissue forces act on staple (790), expansion of staple (790) occurs as described above. While the above examples describe expansion of staple (790), in some instances, hinge feature (795) also allows for staple (790) to move or change orientation from an expanded state to a more retracted or compact state. This could be the case when forces acting on staple (790) subside. Of course, in such examples hinge feature (795) can be configured to allow for expansion only without the ability to contract or close down to a smaller width. In view of the teachings herein, other ways to configure staple (790) with hinge feature (795) will be apparent to those of ordinary skill in the art.

### 5. Exemplary Expandable Staples with "V" Shaped Crown and Cable

FIG. 19 depicts an exemplary staple (890), which can replace staple (190) or (90) in the plurality of staples. Staple (890) includes crown (896) and a pair of legs (898). Crown (896) includes two straight portions (897) and a cable (895). Cable (895) in the present example extends from one leg (898), through straight portions (897), to the other leg (898). In the present example, cable (895) is configured as a unitary cable structure, while in other examples cable feature (895) is configured as multiple discrete sections of cable structure that join or connect two portions of staple (890). In the present example, cable (895) is present where each leg (898) meets or connects with a respective straight portion (897). In some other versions, cable (895) is omitted here such that cable (895) is present only in the middle portion of staple (890) connecting straight portions (897).

In the undeployed state as shown in FIG. 19, staple (890) defines an angle (α6) between straight portions (897), and staple (890) defines a distance (d6) between legs (898). After deployment and after staple (890) is subject to tension from tissue forces as described above, staple (890) expands in width by bending cable (895) such that angle (α6) increases, which also corresponds with an increase in distance (d6). This produces a broader "V" shape for staples (890) that resembles staples (890) becoming closer to linear shaped.

In the present example, cable (895) is configured such that it is operable to change the orientation of staple (890) depending on the forces staple (890) is subjected to. Also in the present example, cable (895) is configurable such that a tension within cable (895) can be such that a threshold amount of force must be imparted upon cable (895) to cause staple (890) to change configuration as described above. For instance, in one version the amount of contact between cable (895) and straight portions (897) through which cable (895) passes can be configured to provide greater or lesser friction among these components, thereby making staple (890) more or less responsive to a given force applied on staple (890). Other ways to configure the tension with cable (895) will be apparent to those of ordinary skill in the art in view of the teachings herein. For instance, the rigidity of cable (895) can be configured based on the material, construction, or diameter of cable (895).

Staple (890) can be considered to define a first portion that includes one leg (898) and one portion (897) of crown (896), and a second portion that includes the other leg (898) and the other portion (897) of crown (896). In this manner, first and second portions of staple (890) are connected by cable (895) and are operable to move relative to one another about cable (895) depending on the forces being exerted on the first and second portions of staple (890). In some instances, a force may be exerted evenly on staple (890) such that each of the first and second portions of staple (890) experience the same force and move relative to one another in a similar fashion. In some other instances, a different degree of force may act on each of the first and second portions of staple (890). In such examples, the degree of movement of the first and the second portions of staple (890) may differ. For example, the portion of staple (890) experiencing the larger force may move a greater distance radially compared to the other portion of staple (890) experiencing the lower force.

As shown in FIG. 19, cable (895) is coplanar with crown (896). Additionally, legs (898) extend generally orthogonal to a plane defined by crown (896) and cable (895). In use after deployment of staples (890), tension from radial tissue forces act on staple (890) and such forces are generally orthogonal to axes defined by legs (898) when in the unformed state. Accordingly, in this manner, cable (895) is configured and oriented so that when radial tissue forces act on staple (890), expansion of staple (890) occurs as described above. While the above examples describe expansion of staple (890), in some instances, cable (895) also allows for staple (890) to move or change orientation from an expanded state to a more retracted or compact state. This could be the case when forces acting on staple (890) subside. Of course, in such examples cable (895) can be configured to allow for expansion only without the ability to contract or close down to a smaller width. In view of the teachings herein, other ways to configure staple (890) with cable (895) will be apparent to those of ordinary skill in the art.

### B. Exemplary Staple Patterns for Staples with "V" Shaped Crowns

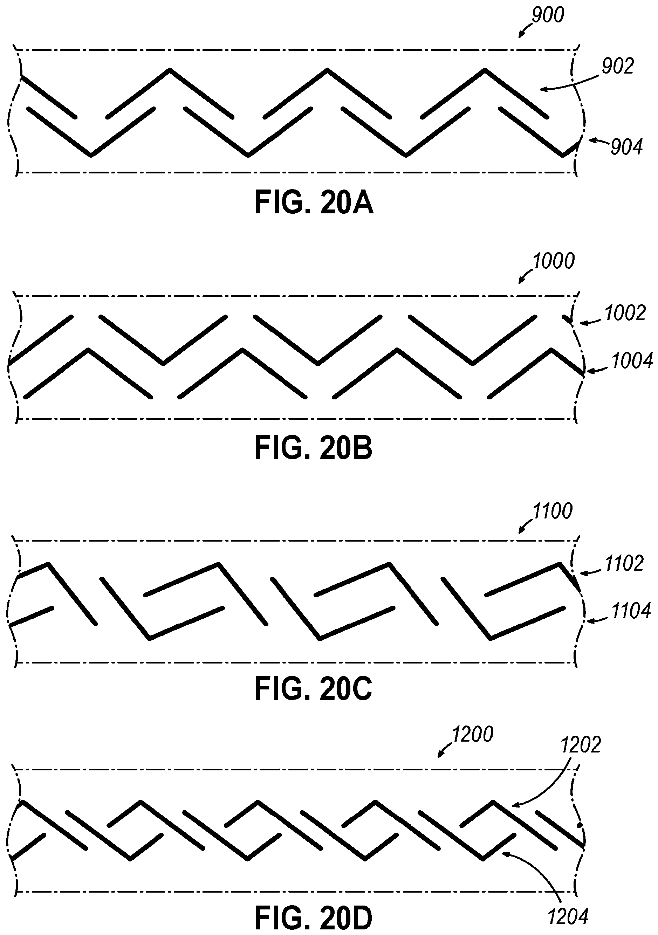

Another variable for consideration with expandable staples is staple pattern. The staple pattern pertains to the arrangement and spacing of the staples. FIGS. 20A-20D illustrate staple patterns that may be used or adapted for use in a circular staple configuration and with expandable staples, such as staples (190, 290, 390, 490, 590, 690, 790, 890) described above.

FIG. 20A illustrates an exemplary staple pattern (900) for "V" shaped staples with equal length arms. As used herein, the "arms" of a "V" shaped staple is understood to refer to the distance along the crown portion from a given staple leg to the vertex. While FIG. 20A depicts staple pattern (900) in a linear pattern, this pattern (900) is adaptable into a wrap-around staple pattern for a circular stapler application. For instance, with a circular stapler, the staples may differ in size and/or shape between the inner row and outer row, although they could be of the same size and/or shape in some versions.

With staple pattern (900), there is a first row or outer row (902) of staples and a second row or inner row (904) of staples. As shown, the staples of first row (902) are opposite facing and offset from the staples of second row (904). This offset appears as a lateral offset in pattern (900), and this offset appears as a circumferential offset when pattern (900) is in a circular or wrap-around staple pattern as seen in FIGS. 8-12. Furthermore, in the present example the opposite facing arrangement is shown with the staples in outer row (902) have the vertex oriented to point outward, while the staples in inner row (904) have the vertex oriented to point inward.

As evident from FIGS. 8-12 and FIG. 20A, with pattern (900), the staples can move from their initial "V" shape to an expanded "V" shape without the staples interfering with one another. As shown in FIGS. 8-12, pattern (900) allows for outward radial expansion of a circular staple pattern when the staples undergo expansion as described above.

FIG. 20B illustrates another exemplary staple pattern (1000) for "V" shaped staples with equal length arms. FIG. 20B depicts staple pattern (1000) as a linear pattern; however, this pattern (1000) is adaptable into a wrap-around staple pattern for a circular stapler application similar to pattern (900) as described above.

With staple pattern (1000), there is a first row or outer row (1002) of staples and a second row or inner row (1004) of staples. As shown, the staples of first row (1002) are opposite facing and offset from the staples of second row (1004). This offset appears as a lateral offset in pattern (1000), and this offset appears as a circumferential offset when pattern (1000) is in a circular or wrap-around staple pattern. Furthermore, in the present example the opposite facing arrangement is shown with the staples in outer row (1002) have the vertex oriented to point inward, while the staples in inner row (1004) have the vertex oriented to point outward. With pattern (1000), the staples can move from their initial "V" shape to an expanded "V" shape without the staples interfering with one another. Furthermore, pattern (1000) allows for outward radial expansion of a circular staple pattern when the staples undergo expansion as described above.

FIG. 20C illustrates another exemplary staple pattern (1100) for "V" shaped staples with equal length arms. FIG. 20C depicts staple pattern (1100) as a linear pattern; however, this pattern (1100) is adaptable into a wrap-around staple pattern for a circular stapler application similar to pattern (900) as described above.

With staple pattern (1100), there is a first row or outer row (1102) of staples and a second row or inner row (1104) of staples. As with pattern (900), with pattern (1100), the staples of outer row (1102) have their vertex generally pointing outward while the staples of inner row (1104) have their vertex generally pointing inward. Moreover, pattern (1100) has staple rows (1102, 1104) with staples that are canted in order to stagger the anchor points between the staple and the tissue to not be parallel to the staple line. This canted arrangement for staples also increases the rigid-body rotation of the staples independent of their flexure. In this manner, pattern (1100) provides for a stapling arrangement with less stress on the tissue for a given amount of elongation of the staple line compared to at least some other stapling patterns. As with the other patterns, with pattern (1100) the staples can move from their initial "V" shape to an expanded "V" shape without the staples interfering with one another. Furthermore, pattern (1100) allows for outward radial expansion of a circular staple pattern when the staples undergo expansion as described above.

FIG. 20D illustrates another exemplary staple pattern (1200) for "V" shaped staples with unequal length arms. FIG. 20D depicts staple pattern (1200) as a linear pattern; however, this pattern (1200) is adaptable into a wrap-around staple pattern for a circular stapler application similar to pattern (900) as described above.

With staple pattern (1200), there is a first row or outer row (1202) of staples and a second row or inner row (1204) of staples. As shown, the staples of first row (1202) are opposite facing and offset from the staples of second row (1204). This offset appears as a lateral offset in pattern (1200), and this offset appears as a circumferential offset when pattern (1200) is in a circular or wrap-around staple pattern. Furthermore, in the present example the opposite facing arrangement is shown with the staples in outer row (1202) have the vertex oriented to point outward, while the staples in inner row (1004) have the vertex oriented to point inward. With pattern (1200), the staples can move from their initial "V" shape to an expanded "V" shape without the staples interfering with one another. Furthermore, pattern (1200) allows for outward radial expansion of a circular staple pattern when the staples undergo expansion as described above.

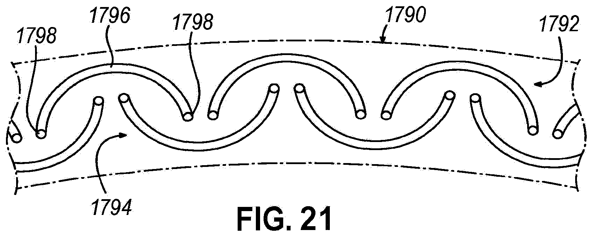

FIG. 21 depicts another exemplary expandable staple (1790). Staples (1790) are configured with "C" shaped crowns (1796) and straight legs (1798). Staples (1790) are configured in a horizontal nested pattern with an outer row (1792) of staples (1790) opening inward toward a center of the circular staple pattern, and an inner row (1794) of staples (1790) opening outward away from a center of the circular staple pattern. In this manner, each "C" shaped staple (1790) overlaps adjacent staples (1790) with the resultant staple pattern eliminating gaps in the circular staple pattern.

FIG. 21 illustrates a relaxed state of the nested arrangement of staples (1790); however, the nested arrangement of staples (1790) allows for expansion of the staple line in response to tissue expansion forces acting on staples (1790). More specifically, as tissue forces act on staples (1790) in their nested arrangement, staples (1790) reorient their relative position such that the nesting arrangement becomes less such that the overlapping of staples (1790) decreases when the pattern expands. Furthermore, in some versions, staples (1790) can also provide for expansion by staples (1790) themselves expanding in similar ways as described above where the "C" shaped crowns (1796) adopt a less curvature shape thereby also increasing their width as described above with respect to other staple versions. In this manner, expansion of the staple line creating the anastomosis using the arrangement shown in FIG. 21 can be achieved in two ways: by the nesting pattern expanding, and/or by staples (1790) themselves expanding.

### III. Miscellaneous

It should also be understood that any one or more of the teachings, examples, etc. described herein may be combined with any one or more of the other teachings, examples, etc. that are described herein. The above-described teachings, examples, etc. should therefore not be viewed in isolation relative to each other. Various suitable ways in which the teachings herein may be combined will be readily apparent to those of ordinary skill in the art in view of the teachings herein.

Some versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California.

The devices described above may be designed to be disposed of after a single use, or they can be designed to be used multiple times. They may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly.

By way of example only, the devices described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Further adaptations of the systems described herein may be accomplished by appropriate modifications by one of ordinary skill in the art without departing from the scope of the present invention. Several of such potential modifications have been mentioned, and others will be apparent to those skilled in the art. For instance, the geometrics, materials, dimensions, ratios, and the like discussed above are illustrative and are not required. Accordingly, the scope of the present invention should be considered in terms of the following claims and is understood not to be limited to the details of structure and operation shown and described in the specification and drawings.

## Claims

1. An apparatus (10) for creating an anastomosis between two lumens, comprising:
(a) a body (100);
(b) a shaft (200) extending distally from the body (100);
(c) a stapling head assembly (300) positioned at a distal end of the shaft (200), wherein the stapling head assembly (200) includes:
(i) an annular deck member (320), wherein the annular deck member (320) includes:
(A) a deck surface (322) configured to contact a tissue defining a select one of the two lumens,
(B) an annular array of staple openings (324, 624) formed through the deck surface (322), wherein the annular array of staple openings (324, 624) is configured with a pattern (900, 1000, 1100, 1200) having two rows (902, 904; 1002, 1004; 1102, 1104; 1202, 1204) of offset openings (324, 624), and
(C) a plurality of staples (190, 1790) configured to travel through the staple openings (324, 624), each staple (190, 1790) of the plurality of staples comprising a crown (196, 296, 396, 496, 596, 696, 796, 896, 1796) and a pair of legs (198, 298, 398, 498, 598, 698, 798, 898, 1798) extending from the crown, wherein the crowns are angled such that each staple (190) defines a "V" shape when viewed from the top or the bottom, or the crown is curved such that each staple (1790) defines a "C" shape when viewed from the top or the bottom, wherein each of the plurality of staples (190, 1790) is expandable having a first configuration and a second configuration, wherein in the second configuration a distance between the pair of legs is greater than in the first configuration;
(ii) a staple driver (352, 652) operable to drive the plurality of staples (190) through the annular array of staple openings (324, 624); and
(d) an anvil (400) configured to deform the staples (190, 1790) driven by the staple driver (352, 652) to form a ring-shaped staple pattern configured to connect the two lumens, wherein the staples (190, 1790) in one row (902; 1002; 1102; 1202) are offset from the staples (190, 1790) in the other row (904; 1004; 1104; 1204);
**characterized in that:**
(e) the "V" shapes or "C" shapes defined by the staples (190, 1790) in one row are oppositely oriented to the "V" shapes or "C" shapes defined by adjacent staples (190, 1790) in the other row.

2. The apparatus (10) of claim 1, when the crowns (396, 496, 596) are angled such that each staple defines a "V" shape when viewed from the top or bottom, and wherein the crown of each staple comprises a weakened portion (395, 495, 595) to promote bending for expandability when the staple (390, 490, 590) is exposed to tension due to tissue forces.

3. The apparatus (10) of claim 2, wherein the weakened portion (395, 495) comprises a reduction in material of the crown (396, 496).

4. The apparatus (10) of claim 3, wherein the reduction in material of the crown (396) consists of a notch in the crown that connects with remaining portions of the crown (396) that are cylindrically shaped.

5. The apparatus (10) of claim 3, wherein the reduction in material of the crown (496) consists of a portion having a reduced diameter that connects with remaining portions of the crown (496) that are cylindrically shaped.

6. The apparatus (10) of claim 3, wherein the reduction in material of the crown (395) consists of a portion (395) having a flat area that connects with remaining portions of the crown (395) that are cylindrically shaped.

7. The apparatus of claim 1, when the crown (10) of each staple (190) of the plurality of staples is angled such that each staple defines a "V" shape when viewed from the top or bottom, and wherein the crown of each staple of the plurality of staples comprises a spring feature (295) comprising a bend in the crown (296) configured to straighten, wherein the spring feature (295) provides for automatic expansion of each of the staples (290) after the staples (290) are deployed.

8. The apparatus (10) of any one of claim 1 or claim 2, when the crown (696, 796) of each staple (690, 790) of the plurality of staples is angled such that each staple defines a "V" shape when viewed from the top or bottom, and wherein the crown of each staple of the plurality of staples comprises a hinge feature (695, 795), wherein the hinge feature (695, 795) promotes adjustment of the staple (690, 790) for expandability when the staple (690, 790) is exposed to tension due to tissue forces.

9. The apparatus (10) of claim 8, wherein the hinge feature (695) consists of an eyelet (691, 692) connected with two straight portions of the crown.

10. The apparatus (10) of claim 8, wherein the hinge (795) feature consists of a pair of eyelets (791, 792) spaced apart and connected by a connecting member (793), wherein each eyelet of the pair of eyelets (791, 792) connects with a separate straight portion of the crown.

11. The apparatus (10) of claim 1 or claim 2, wherein each staple (890) of the plurality of staples comprises a flexible cable (895) connecting portions of the crown, when the crown of each staple is angled such that each staple defines a "V" shape when viewed from the top or bottom.

## Patentansprüche

1. Einrichtung (10) zum Erzeugen einer Anastomose zwischen zwei Lumen, umfassend:
(a) einen Körper (100);
(b) einen Schaft (200), der sich von dem Körper (100) distal erstreckt;
(c) eine Klammerkopfbaugruppe (300), die an einem distalen Ende des Schafts (200) positioniert ist,
wobei die Klammerkopfbaugruppe (200) einschließt:
(i) ein ringförmiges Deckelement (320), wobei das ringförmige Deckelement (320) einschließt:
(A) eine Deckoberfläche (322), die konfiguriert ist, um ein Gewebe zu kontaktieren, das ein ausgewähltes der zwei Lumen definiert,
(B) eine ringförmige Anordnung von Klammeröffnungen (324, 624), die durch die Deckoberfläche (322) ausgebildet ist, wobei die ringförmige Anordnung von Klammeröffnungen (324, 624) mit einem Muster (900, 1000, 1100, 1200) konfiguriert ist, das zwei Reihen (902, 904; 1002, 1004; 1102, 1104; 1202, 1204) von versetzten Öffnungen (324, 624) aufweist, und
(C) eine Vielzahl von Klammern (190, 1790), die konfiguriert ist, um sich durch die Klammeröffnungen (324, 624) zu bewegen, jede Klammer (190, 1790) der Vielzahl von Klammern umfassend eine Krone (196, 296, 396, 496, 596, 696, 796, 896, 1796) und ein Paar von Schenkeln (198, 298, 398, 498, 598, 698, 798, 898, 1798), die sich von der Krone erstrecken, wobei die Kronen derart schräg verlaufend sind, dass jede Klammer (190) eine "V"-Form definiert, wenn sie von oben oder unten betrachtet wird, oder die Krone derart gekrümmt ist, dass jede Klammer (1790) eine "C"-Form definiert, wenn sie von oben oder unten betrachtet wird, wobei jede der Vielzahl von Klammern (190, 1790) erweiterbar ist und eine erste Konfiguration und eine zweite Konfiguration aufweist, wobei in der zweiten Konfiguration ein Abstand zwischen dem Paar von Schenkeln größer als in der ersten Konfiguration ist;
(ii) einen Klammertreiber (352, 652), der betätigbar ist, um die Vielzahl von Klammern (190) durch die ringförmige Anordnung von Klammeröffnungen (324, 624) hindurch zu treiben; und
(d) einen Amboss (400), der konfiguriert ist, um die Klammern (190, 1790) zu verformen, die durch den Klammertreiber (352, 652) angetrieben werden, um ein ringförmiges Klammermuster auszubilden, das konfiguriert ist, um die zwei Lumen zu verbinden, wobei die Klammern (190, 1790) in einer Reihe (902; 1002; 1102; 1202) zu den Klammern (190, 1790) in der anderen Reihe (904; 1004; 1104; 1204) versetzt sind;
**dadurch gekennzeichnet, dass**:
(e) die "V"-Formen oder "C"-Formen, die durch die Klammern (190, 1790) in einer Reihe definiert sind, entgegengesetzt zu den "V"-Formen oder "C"-Formen ausgerichtet sind, die durch angrenzende Klammern (190, 1790) in der anderen Reihe definiert sind.

2. Einrichtung (10) nach Anspruch 1, wenn die Kronen (396, 496, 596) derart schräg verlaufend sind, dass jede Klammer, wenn sie von oben oder unten betrachtet wird, eine "V"-Form definiert, und wobei die Krone jeder Klammer einen geschwächten Abschnitt (395, 495, 595) umfasst, um die Biegung für eine Erweiterbarkeit zu fördern, wenn die Klammer (390, 490, 590) aufgrund von Gewebekräften einer Spannung ausgesetzt ist.

3. Einrichtung (10) nach Anspruch 2, wobei der geschwächte Abschnitt (395, 495) eine Materialreduzierung der Krone (396, 496) umfasst.

4. Einrichtung (10) nach Anspruch 3, wobei die Materialreduzierung der Krone (396) aus einer Kerbe in der Krone besteht, die mit verbleibenden Abschnitten der Krone (396) verbunden ist, die zylindrisch geformt sind.

5. Einrichtung (10) nach Anspruch 3, wobei die Materialreduzierung der Krone (496) aus einem Abschnitt besteht, der einen reduzierten Durchmesser aufweist, der mit verbleibenden Abschnitten der Krone (496) verbunden ist, die zylindrisch geformt sind.

6. Einrichtung (10) nach Anspruch 3, wobei die Materialreduzierung der Krone (395) aus einem Abschnitt (395) besteht, der einen flachen Bereich aufweist, der sich mit verbleibenden Abschnitten der Krone (395) verbindet, die zylindrisch geformt sind.

7. Einrichtung nach Anspruch 1, wenn die Krone (10) jeder Klammer (190) der Vielzahl von Klammern derart schräg verlaufend ist, dass jede Klammer eine "V"-Form definiert, wenn sie von oben oder unten betrachtet wird, und wobei die Krone jeder Klammer der Vielzahl von Klammern ein Federmerkmal (295) umfasst, umfassend eine Biegung in der Krone (296), die konfiguriert ist, um sich zu begradigen, wobei das Federmerkmal (295) für eine automatische Erweiterung jeder der Klammern (290) sorgt, nachdem die Klammern (290) eingesetzt wurden.

8. Einrichtung (10) nach einem von Anspruch 1 oder 2, wenn die Krone (696, 796) jeder Klammer (690, 790) der Vielzahl von Klammern derart schräg verlaufend ist, dass jede Klammer, wenn sie von oben oder unten betrachtet wird, eine "V"-Form definiert, und wobei die Krone jeder Klammer der Vielzahl von Klammern ein Scharniermerkmal (695, 795) umfasst, wobei das Scharniermerkmal (695, 795) eine Anpassung der Klammer (690, 790) für die Erweiterbarkeit fördert, wenn die Klammer (690, 790) aufgrund von Gewebekräften der Spannung ausgesetzt ist.

9. Einrichtung (10) nach Anspruch 8, wobei das Scharniermerkmal (695) aus einer Öse (691, 692) besteht, die mit zwei geraden Abschnitten der Krone verbunden ist.

10. Einrichtung (10) nach Anspruch 8, wobei das Scharnier(795)-Merkmal aus einem Paar Ösen (791, 792) besteht, die beabstandet und durch ein Verbindungselement (793) verbunden sind, wobei jede Öse des Paares von Ösen (791, 792) mit einem separaten geraden Abschnitt der Krone verbunden ist.

11. Einrichtung (10) nach Anspruch 1 oder 2, wobei jede Klammer (890) der Vielzahl von Klammern ein flexibles Kabel (895) umfasst, das Abschnitte der Krone verbindet, wenn die Krone jeder Klammer derart schräg verlaufend ist, dass jede Klammer eine "V"-Form definiert, wenn sie von oben oder unten betrachtet wird.

## Revendications

1. Appareil (10) pour la création d'une anastomose entre deux lumières, comprenant :
(a) un corps (100) ;
(b) une tige (200) s'étendant distalement à partir du corps (100) ;
(c) un ensemble tête d'agrafage (300) positionné au niveau d'une extrémité distale de la tige (200), dans lequel l'ensemble tête d'agrafage (200) comporte :
(i) un organe formant pont annulaire (320), dans lequel l'organe formant pont annulaire (320) comporte :
(A) une surface de pont (322) conçue pour venir en contact avec un tissu définissant une lumière sélectionnée parmi les deux lumières,
(B) un réseau annulaire d'ouvertures pour agrafes (324, 624) formé à travers la surface de pont (322), dans lequel le réseau annulaire d'ouvertures pour agrafes (324, 624) est conçu avec un motif (900, 1000, 1100, 1200) ayant deux rangées (902, 904 ; 1002, 1004 ; 1102, 1104 ; 1202, 1204) d'ouvertures décalées (324, 624), et
(C) une pluralité d'agrafes (190, 1790) conçues pour se déplacer à travers les ouvertures pour agrafes (324, 624), chaque agrafe (190, 1790) de la pluralité d'agrafes comprenant une couronne (196, 296, 396, 496, 596, 696, 796, 896, 1796) et une paire de pattes (198, 298, 398, 498, 598, 698, 798, 898, 1798) s'étendant à partir de la couronne, dans lequel les couronnes sont inclinées de telle sorte que chaque agrafe (190) définit une forme en « V » lorsqu'elle est vue de dessus ou de dessous, ou la couronne est incurvée de telle sorte que chaque agrafe (1790) définit une forme en « C » lorsqu'elle est vue de dessus ou de dessous, dans lequel chacune parmi la pluralité d'agrafes (190, 1790) est expansible en ayant une première configuration et une seconde configuration, dans lequel, dans la seconde configuration, une distance entre la paire de pattes est supérieure à celle dans la première configuration ;
(ii) un entraînement d'agrafes (352, 652) actionnable pour entraîner la pluralité d'agrafes (190) à travers le réseau annulaire d'ouvertures pour agrafes (324, 624) ; et
(d) une enclume (400) conçue pour déformer les agrafes (190, 1790) entraînées par l'entraînement d'agrafes (352, 652) afin de former un motif d'agrafes de forme annulaire conçu pour relier les deux lumières, dans lequel les agrafes (190, 1790) dans une rangée (902 ; 1002 ; 1102 ; 1202) sont décalées par rapport aux agrafes (190, 1790) dans l'autre rangée (904 ; 1004 ; 1104 ; 1204) ;
**caractérisée en ce que :**
(e) les formes en « V » ou les formes en « C » définies par les agrafes (190, 1790) dans une rangée sont orientées de manière opposée par rapport aux formes en « V » ou aux formes en « C » définies par des agrafes (190, 1790) adjacentes dans l'autre rangée.

2. Appareil (10) selon la revendication 1, lorsque les couronnes (396, 496, 596) sont inclinées de telle sorte que chaque agrafe définit une forme en « V » lorsqu'elle est vue de dessus ou de dessous, et dans lequel la couronne de chaque agrafe comprend une partie affaiblie (395, 495, 595) pour favoriser la flexion à des fins d'expansibilité lorsque l'agrafe (390, 490, 590) est exposée à une tension due aux forces tissulaires.

3. Appareil (10) selon la revendication 2, dans lequel la partie affaiblie (395, 495) comprend une réduction de matière de la couronne (396, 496).

4. Appareil (10) selon la revendication 3, dans lequel la réduction de matière de la couronne (396) consiste en une encoche dans la couronne qui est reliée aux parties restantes de la couronne (396) qui sont de forme cylindrique.

5. Appareil (10) selon la revendication 3, dans lequel la réduction de matière de la couronne (496) consiste en une partie ayant un diamètre réduit qui est reliée aux parties restantes de la couronne (496) qui sont de forme cylindrique.

6. Appareil (10) selon la revendication 3, dans lequel la réduction de matière de la couronne (395) consiste en une partie (395) ayant une zone plate qui est reliée aux parties restantes de la couronne (395) qui sont de forme cylindrique.

7. Appareil selon la revendication 1, lorsque la couronne (10) de chaque agrafe (190) de la pluralité d'agrafes est inclinée de telle sorte que chaque agrafe définit une forme en « V » lorsqu'elle est vue de dessus ou de dessous, et dans lequel la couronne de chaque agrafe de la pluralité d'agrafes comprend un élément à ressort (295) comprenant un coude dans la couronne (296) conçu pour se redresser, dans lequel l'élément à ressort (295) permet l'expansion automatique de chacune des agrafes (290) après que les agrafes (290) ont été déployées.

8. Appareil (10) selon l'une quelconque de la revendication 1 ou de la revendication 2, dans lequel la couronne (696, 796) de chaque agrafe (690, 790) de la pluralité d'agrafes est inclinée de telle sorte que chaque agrafe définit une forme en « V » lorsqu'elle est vue de dessus ou de dessous, et dans lequel la couronne de chaque agrafe de la pluralité d'agrafes comprend un élément formant charnière (695, 795), dans lequel l'élément formant charnière (695, 795) favorise l'ajustement de l'agrafe (690, 790) à des fins d'extensibilité lorsque l'agrafe (690, 790) est exposée à une tension due aux forces tissulaires.

9. Appareil (10) selon la revendication 8, dans lequel l'élément formant charnière (695) consiste en un œillet (691, 692) relié à deux parties droites de la couronne.

10. Appareil (10) selon la revendication 8, dans lequel l'élément formant charnière (795) consiste en une paire d'œillets (791, 792) espacés et reliés par un organe de liaison (793), dans lequel chaque œillet de la paire d'œillets (791, 792) est relié à une partie droite distincte de la couronne.

11. Appareil (10) selon la revendication 1 ou la revendication 2, dans lequel chaque agrafe (890) de la pluralité d'agrafes comprend un câble flexible (895) reliant des parties de la couronne, lorsque la couronne de chaque agrafe est inclinée de telle sorte que chaque agrafe définit une forme en « V » lorsqu'elle est vue de dessus ou de dessous.
